# EUROPEAN PATENT SPECIFICATION

(11) **EP 2 536 361 B1**
(45) Date of publication and mention of the grant of the patent: **29.07.2015**
(21) Application number: 11721822.2
(22) Date of filing: 19.02.2011
(51) Int. Cl.: A61F 2/30, A61F 2/28, A61C 8/00

(54) **METHOD AND DEVICE FOR FABRICATING A PATIENT-SPECIFIC IMPLANT**
VERFAHREN UND VORRICHTUNG ZUR HERSTELLUNG EINES PATIENTENSPEZIFISCHEN IMPLANTATS
PROCÉDÉ ET DISPOSITIF DE FABRICATION D'UN IMPLANT PROPRE À UN PATIENT

(30) Priority: 19.02.2010 US 305973 P
(43) Date of publication of application: 26.12.2012
(73) Proprietor: Inspire AG IRPD, 9014 St. Gallen (CH)
(72) Inventor: SCHINDEL, Ralf, 9011 St. Gallen (CH); BAUER, Ronald, 9053 Teufen (CH); ARTUSI, Reto, 8248 Uhwiesen (CH)
(74) Representative: Chaillot, Geneviève
(86) International application number: PCT/IB2011/000330
(87) International publication number: WO 2011/101731

(56) References cited:
- US-A- 5 397 361
- US-A1- 2004 029 075
- US-A1- 2006 224 242
- US-A1- 2007 198 022

## Description

This invention relates to a device in the form of a kit, a method of making the kit, and a method of use of the kit for the fabrication of a patient-specific implant, particularly a hard implant such as a bone implant of a skull fragment, a hip or other bone fragment.

### BACKGROUND OF THE INVENTION

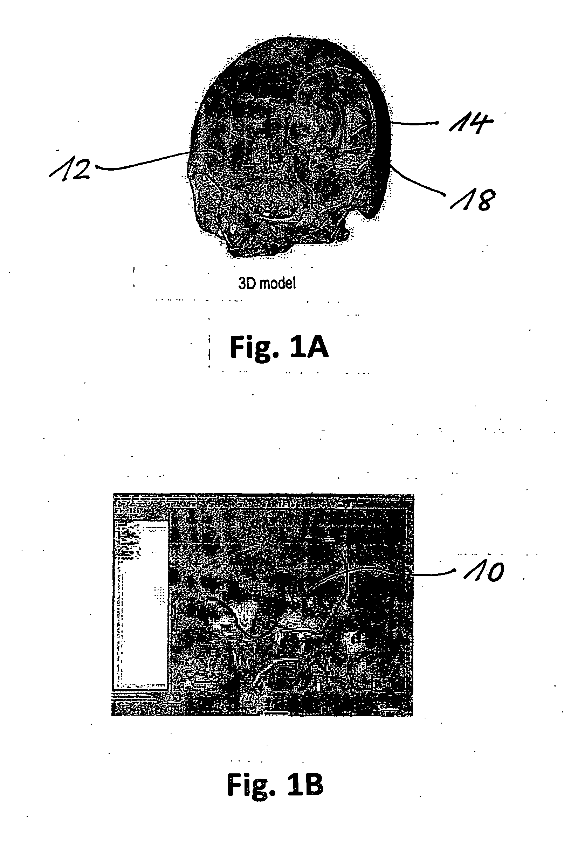

Referring to **FIGs. 1A and 1B**, in typical neurosurgical operations on the brain, a cranial calotte or calvarial fragment 10 must be removed, in other words, a portion of the skull 12 will be removed, creating an opening 14, to provide access to a ventricle of the skull. In order to close this opening after the operation, it is not possible to simply re-insert the removed fragment 12, largely because swelling of the brain 10 occurs usually after brain surgery and such fragment, if reinserted, would cause unacceptable pressure on the brain. Additionally the geometry of the removed fragment is smaller than the opening in the size of the milling cleft. Therefore, the scalp is closed without reinserting the removed fragment 12 and the patient's head is often protected with an external, removable prosthesis (not shown).

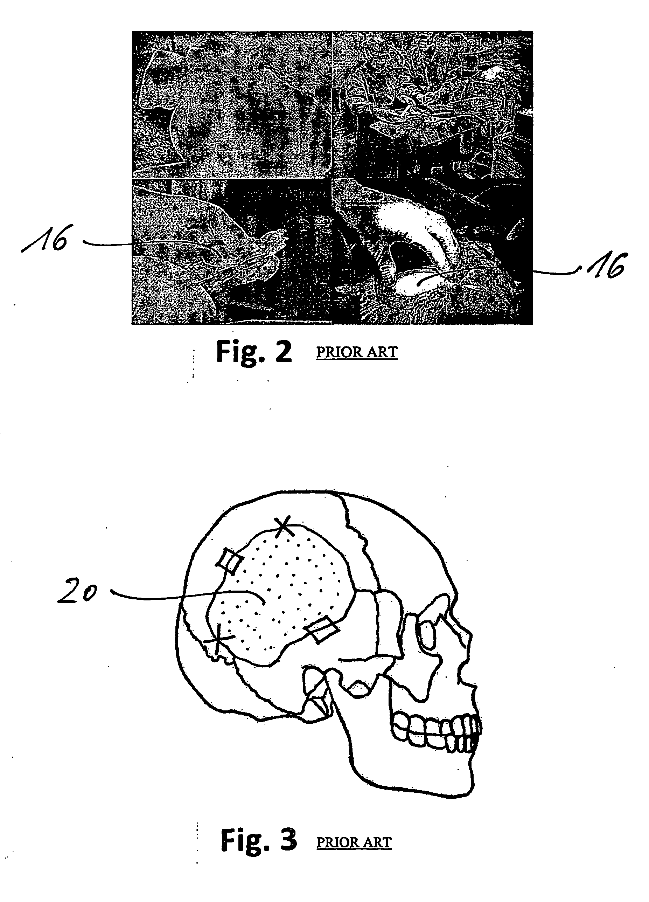

Referring now to **FIG. 2****,** according to the state of the art practiced today, the standard procedure is to wait from two to six months after the operation before inserting a hand-formed, patient-specific prosthesis 16 under the scalp or skin which will close the opening made during surgery. This prosthesis 16 is generally formed during the operation to repair the opening from a two component bone cement such as PMMA (Polymethylmethacrylate), which is kneaded and hand-formed directly on the dura mater 18, the outer soft tissue covering of the brain. Then it must be allowed to harden for between ten and twenty minutes.

The disadvantage of this prior art method is that the time required to fabricate the replacement calotte or fragment 16 together with the set time of the bone cement (during which fabrication the surgeon is not directly focused on the patient but rather on the creation of the prosthesis), the surgeons, nurses and staff are occupied, and the patient has an open wound in his head, and is thus susceptible to infection.

A further disadvantage is that corrections to the geometric form of the replacement fragment 16 are difficult to make, sometimes requiring that the surgeon and his staff restart the process of fabrication of the replacement fragment again, thus further delaying the operation, costing more time and thus money, and occupying surgical infrastructure. The quality of the replacement fragment 16 can depend on the level of experience and fatigue of the surgeon, thus adding risks to the surgical procedure.

A further disadvantage is that to receive the correct and fitting shape of the fragment the surgeon should be able to knead the bone cement with pressure against the bone and dura mater tissue. But giving preasure on the dura mater is allowed only very limited.

Referring now to **FIG. 3****,** a further prior art method for the fabrication of a replacement fragment 20 is a pre-operative method which uses computer tomography data (CT/MRI-data). With this data, a suitable patient-specific replacement fragment 20 can be engineered and direct milled out of titanium or PEEK (an advanced plastics material, Polyetheretherketone). This process is only used where very complex bone geometries must be replaced, because of the costs associated with the fabrication of expensive geometries using this process, as well as the high material costs associated with the use of PEEK plastics approved for medical uses. Further, there is practically no opportunity to modify the geometry intra operatively, given by the toughness and hardness of the material used. Thus, this prosthesis 20 must fit or the operation is aborted and has to be rescheduled for another attempt.

US 5,397,361 forms the basis for the preamble of claim 1. It discloses a method and kit for producing a cranial plate duplicating the dimensions of an excised portion of the cranium. The kit comprises a sterile impression material used for making an impression of an excised portion of a patient's skull, a small amount of sterile water usable with the impression material, three small mixing bowls and two small mixing spatulas. The method comprises preparing a three-dimensional impression of the excised portion in the impression material, removing the excised portion from the impression material to provide a mold cavity, filling the mold cavity with a sterile resinous material which sets, removing the set resinous material to form a plate duplicating the dimensions of the excised portion, and finally surgically installing the plate in the patient's cranium to replace the excised portion earlier removed.

US 2006/0224242, US 2004/0029075 and US 2007/0198022 disclose similar known methods and devices.

What is needed is a method which avoids the significant disadvantages of the prior art solutions, and to provide the surgeon with a means by which he or she can quickly and inexpensively fabricate a patient-specific replacement calotte fragment 16, thereby saving time and resources, and subjecting the patient to less risk of infection or other complications that may be caused by a longer surgical procedure.

### SUMMARY OF THE INVENTION

An object of the invention is to minimize the time required to perform a surgical intervention, particularly the replacement of a three dimensional hard body part fragment, such as a bone fragment, with a prosthesis.

Another object of the invention is to minimize distraction of a surgeon's attention from the patient while the surgeon is intra-operatively forming a replacement fragment, such as a cranial fragment, and to minimize or eliminate the down-time associated with hardening or setting of the bone cement from which the replacement fragment is formed.

Another object of the invention is to minimize the time and costs associated with a neurosurgical operation.

A kit, a method of fabrication of the kit and a method of use of the kit for facilitating the fabrication of a patient-specific cranial implant is provided which meets the needs identified above.

According to a first aspect of the present invention, there is provided a kit comprising a base body which has a sterilized mould recess for receiving and shaping a not yet cross-linked and/or not yet hardened kneadable mass; and a removable sealing element, characterized in that said mould recess has a patient-specific three-dimensional negative surface contour of at least a part of a patient-specific body part structure such as bone, cartilage or teeth; and said removable sealing element hermetically seals said sterilized mould recess.

This base body with its mould recess enables the surgeon to hand-shape the patent-specific implant by pressing the not yet cross-linked and/or not yet hardened kneadable mass against the mould surface of the mould recess. This guarantees that, one the one hand, at least a first part of the surface of the implant, i.e. the implant surface part interfacing or interacting with the implant recipient's body, is shaped according to the stored patient-specific 3D data, and that, on the other hand, a second part of the surface of the implant, i.e. the implant surface part not interfacing or interacting with the implant recipient's body, will be shaped according the surgeon's hand kneading and/or finger kneading of the not yet cross-linked and/or not yet hardened mass, thus allowing the surgeon to account for practical surgical requirements.

It should be noted that the present invention allows the implant to be made from any biologically acceptable kneadable material which after kneading and shaping will cross-link and/or harden. Depending on the material, such cross-linking and/or hardening will result in an implantable material with mechanical properties ranging from rubber-like to bone-like or even stone-like.

Preferably, the kit's base body has a sterilized kneading recess for receiving said not yet hardened and/or cross-linked mass to be kneaded; and a removable sealing element hermetically sealing said sterilized kneading recess.

This allows the base body to be used both for kneading in the kneading recess and for shaping in the mould recess.

It should be noted that, with some implant materials, it is possible and appreciated by surgeons that the kneaded and shaped implant material may be removed from the mould recess before complete hardening. This may facilitate fitting the implant to the implant recipient's body.

The base body may have a sterilized storage recess in which a first sterilized kneadable mass component and a second sterilized kneadable mass component for preparing said kneadable mass are provided separately from each other; and a removable sealing element hermetically sealing said sterilized storage recess. Preferably, the base body also has a sterilized storage recess in which a sterilized mixing and/or kneading tool for mixing and/or kneading said not yet cross-linked or not yet hardened mass is provided; and a sealing element hermetically sealing said sterilized storage recess.

This enables all the steps of preparing the mass (mixing, kneading) and shaping the mass in the mould recess to be performed on the base body in a sterilized environment.

In a particularly preferred embodiment of the kit, the base body is a thermoformed sheet.

A sealing element may be a foil removably attached to said base body and hermetically sealing the recesses of said base body, or it may be a hermetically sealed bag enclosing said base body.

Advantageously, at least the sterilized surface inside said mould recess and preferably inside said kneading recess is made of an apolar polymer, or said thermoformed sheet is made of an apolar polymer. Such apolar polymer is preferably a C and H based polymer, typically an olefinic or an aromatic polymer, such as polypropylene (PP), polyethylene (PE) or polystyrene (PS).

This prevents unwanted chemical interaction ("sticking") between the mould surface and some preferred and commonly used kneadable masses such as polymethylmethacrylate (PMMA).

According to a second aspect of the present invention, there is provided a method of fabricating a kit as defined in any one of the preceding paragraphs, comprising the following steps:
a) three-dimensionally scanning a patient's body part structure which is to be at least partially replaced and storing said tomographical data;
b) manufacturing a base body having at least one recess with a patient-specific three-dimensional negative surface contour based on said tomographical data;
c) sterilizing the at least one recess of said base body and hermetically sealing it with a removable sealing element.

In a first preferred embodiment of the method of fabricating the kit, step b) consists of manufacturing said base body using an additive (layer-by-layer) process such as laser sintering and providing said base body as the base body of the kit.

In a second preferred embodiment of the method of fabricating the kit, step b) comprises a step b1) for manufacturing an intermeidate body using an additive (layer-by-layer) process such as laser sintering; and a step b2) for using said intermediate body as a mould in a thermoforming process such as deep-drawing for thermoforming a sheet material into a thermoformed sheet and providing said thermoformed sheet as the base body of the kit.

According to a third aspect of the present invention, there is provided a method of using a kit as defined in any one of the preceding paragraphs, comprising the following steps:
- removing the sealing element from the base body in a sterilized environment, thus exposing the sterilized mould recess;
- preparing a kneadable and cross-linkable and/or hardenable (settable) mass;
- placing the prepared mass into the sterilized mould recess, thus shaping at least a part of the surface of said mass in accordance with a patient-specific body part structure;
- allowing the shaped mass to at least partially cross-link and/or harden; and
- removing the at least partially cross-linked and/or hardened shaped mass from said recess.

The mould may be used several times, provided that all the handling takes place in a sterile environment. Also, the surgeon may remove the implant mass before it has completely hardened. This allows the surgeon to make minor adaptations to the shape of the implant mass before or during the fitting of the mass to the implant recipent's body.

Preferably, the kit includes a thermo-formed plastic sheet, formed as a blister pack, to the three dimensional contour of a bone structure of a specific patient typically formed of PP (Polypropylene) plastic (although any other suitable plastic, such as PC, could be used). The same thermo-formed sheet optionally doubles as a container for the remaining elements of the kit, which include bone cement (see http://en.wikipedia.org/wiki/Bone_cement, the contents of which are incorporated herein by reference, referring to bone cement available in two part form, each part contained in a separate container tube), and, optionally, disposable forming aids such as a spatula or a mixing cup. A sealing sheet may be adhered to and extended between flanges of the thermo-formed sheet, to seal the components of the kit within the kit container.

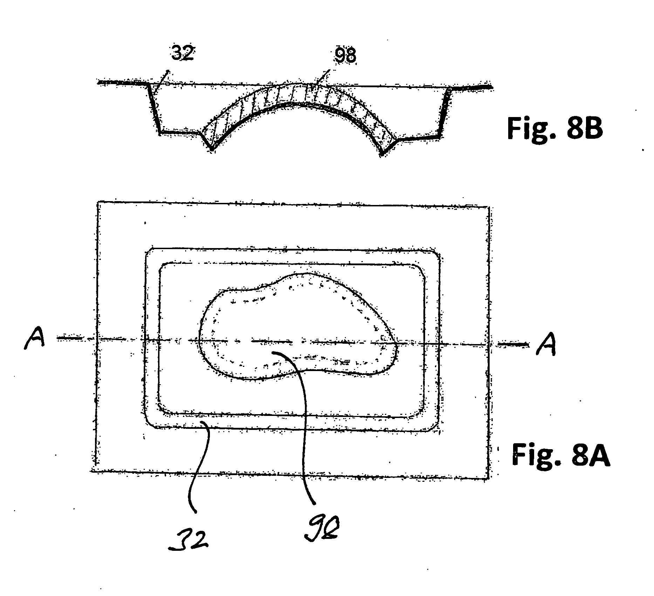

Preferably, the main kit is a thermo-formed sealed packed sterilized plastic sheet which contains the engineered negative form of the patient-specific calotte (FIG 8A and 8B).

This kit could be enlarged or added by a patient-specific model of the wound or bone contour to control geometry of the set PMMA implant.

Preferably, the method of fabrication of the kit includes several steps. In a first fabrication step, CT/MRI data of a patient is created using a CT or MRI scanner. In a second fabrication step, the patient-specific skull fragment is engineered from CT/MRI data, including inputs and modifications requested by the surgeon (e.g. consideration of muscle placement which may change the geometry of the fragment, etc). In a third fabrication step, the deep draw form is engineered through use of 3D data of the form of the patient-specific fragment. Optionally, recesses for accommodation of standard containers of bone cement, a spatula or mixer, are included in the data so as to create a blister pack kit including all components and tools needed to complete the operation. In a fourth fabrication step, the custom mould to thermo form the plastic blister is fabricated by, for example, laser sintering. Optionally, this mould is made as an insert to a thermoforming mold which includes a form for creating the above-mentioned recesses for accommodation of standard containers of bone cement, a spatula or mixer. In a fifth fabrication step, the kneading form is fabricated through, e.g., thermoforming a PP sheet, optionally, together with additional compartments for other components needed to complete the operation. In a sixth fabrication step, the kneading form is optionally filled with the additional components, and at least selectively sterilized by sterilizing and sealing the PP kneading form (partial or one-sided mould).

Preferably, the method of use of the kit includes several steps. In a first step, the kit is opened, exposing the patient-specific thermoformed form. In a second step, the two components of the bone cement (PMMA) are kneaded together to the desired consistency. In a third step, the kneaded bone cement is placed over the patient-specific thermoformed form in order to form the implant. In a fourth step, the bone cement is allowed to set.
In a fifth step, the formed implant is placed on the skull of the patient in a position where it will be fixed. Note that to aid in positioning where the hole is a circular hole formed with core drill bits, a notch, formed on the hole, may be mirrored in the patient-specific PMMA implant. In a sixth step, the surgeon checks if the implant properly fits in place. If not, in a seventh step, the surgeon evaluates whether to attempt to modify the replacement fragment. In an eighth step, if the surgeon cannot make satisfactory modifications, then the method returns to the first step, and a new fragment is made. In a ninth step, the implant is fixed in place, thus closing the wound.

Note that if there is a problem with the fit, the surgeon evaluates whether to attempt to modify the replacement fragment. If the surgeon cannot make satisfactory modifications, then the method is reinitiated, with a new implant being made using the same mould (taking advantage of the fact that the mould is reusable), and returns to the first step, and a new fragment is made and subsequently fixed in place.

### BRIEF DESCRIPTION OF THE DRAWINGS

**FIGs. 1A** and **1B** are perspective views of a skull and in particular, the opening and patient-specific implant whose fabrication is the object of the invention.
**FIG. 2** is a collage of views showing steps of a method of the prior art.
**FIG. 3** is a perspective view of an implant made according to an alternate method of the prior art.
**FIG. 4A** is a top view of a kit of the invention.
**FIG. 4B** is a cross sectional view of a kit of the invention, taken along line A-A of **FIG. 4A****.**
**FIG. 5A** is a flow chart of the method of fabrication of the kit of the invention.
**FIG. 5B** is a schematic view of CT/MRI data capture of a patient's skull.
**FIG. 5C** is a perspective view of a custom (i.e., patient-specific) deep draw form (mould) fabricated by, for example, laser sintering.
**FIG. 5D** is a schematic view of a laser sintering process practiced by Inspire AG, Switzerland.
**FIG. 5E** in a schematic diagram of a typical thermoforming process used to shape the PP form (one-sided mould) of the invention.
**FIGs. 6A** and **6B** are perspective views of typical thermoformed PP forms (one-sided moulds).
**FIG. 7** is a flow chart of the method of use of the kit of the invention.
**FIGs. 8A** and **8B** are top and cross-sectional side views of the thermoformed form / mould of the invention.

Those skilled in the art will appreciate that elements in the Figures are illustrated for simplicity and clarity and have not necessarily been drawn to scale. For example, dimensions may be exaggerated relative to other elements to help improve understanding of the invention and its embodiments. Furthermore, when the terms 'first', 'second', and the like are used herein, their use is intended for distinguishing between similar elements and not necessarily for describing a sequential or chronological order. Those skilled in the art will therefore understand that such terms may be interchangeable with other terms, and that the embodiments described herein are capable of operating in other orientations than those explicitly illustrated or described.

### DETAILED DESCRIPTION OF THE PREFERRED EMBODIMENT

The following description is not intended to limit the scope of the invention in any way as they are exemplary in nature and serve to describe the best mode of the invention known the inventors as of the filing date hereof. Consequently, changes may be made in the arrangement and/or function of any of the elements described in the disclosed exemplary embodiments without departing from the spirit and scope of the invention.

In the instant application, a kit facilitating an implant operation, a method of fabrication of the kit and a method of use of the kit for facilitating the fabrication of a patient-specific cranial implant is provided which reduces the time required for preparation of such implant, increases the focus of the surgeon on the patient and minimizes the time which the patient is exposed to possible infection. The kit provides a means for pre-operative preparation of the implant, thereby eliminating intraoperative preparation and, consequently, eliminating the wait time associated with the setting of the bone cement.

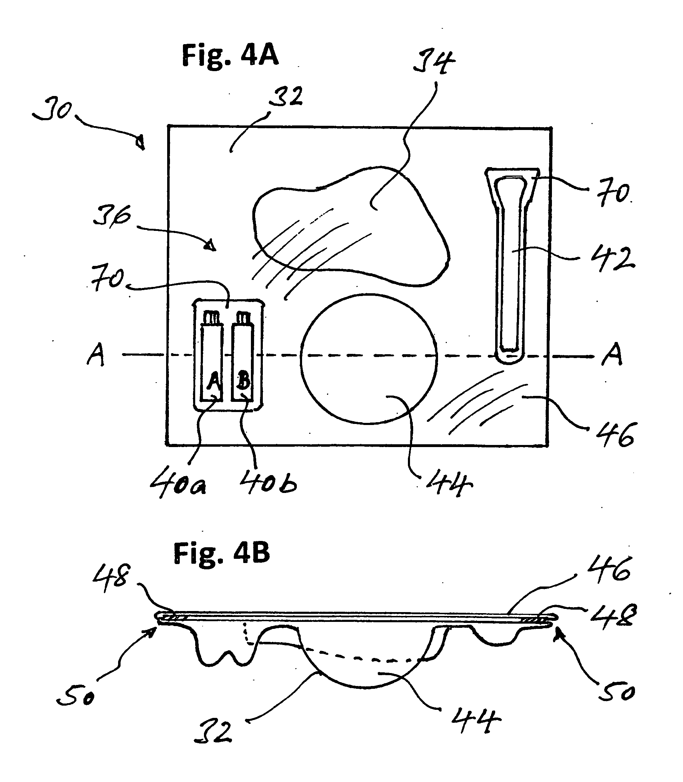

Referring now to **FIGs. 4A** and **4B****,** the kit 30 includes a thermo-formed plastic sheet 32, a portion 34 of which is formed (typically of PP plastics) to the three dimensional contour of a bone structure of a specific patient. The thermo-formed sheet 32 optionally doubles as a container 36 for the remaining elements of the kit, which include bone cement 40a, 40b (typically in two part form, each part contained in a separate container device), and, optionally, disposable forming and/or mixing aids 42 such as a spatula. A kneading recess 44 may also be formed in the thermoformed sheet 32 of the kit 30. A sheet 46 may be adhered to (e.g., via adhesive 48) and extends between flanges 50 of the thermo-formed sheet, to seal and keep sterilized the components 40a, 40b and 42 and the mould recess or shaping form 34 of the kit 30 within the container 36.

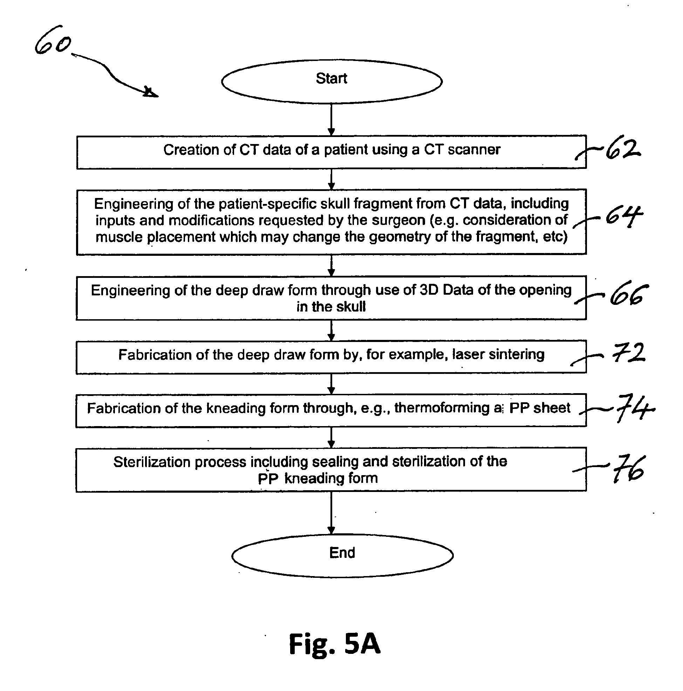

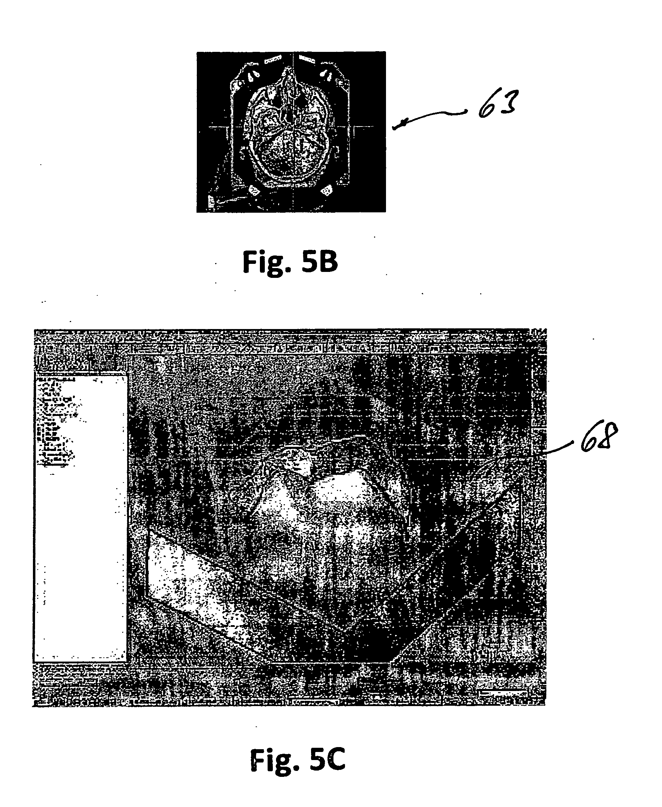

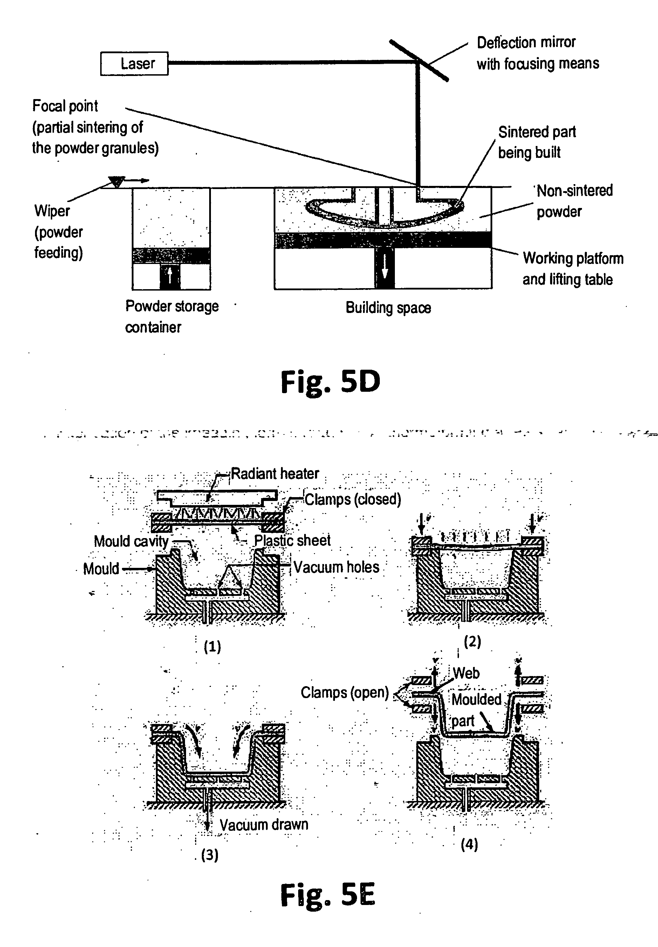

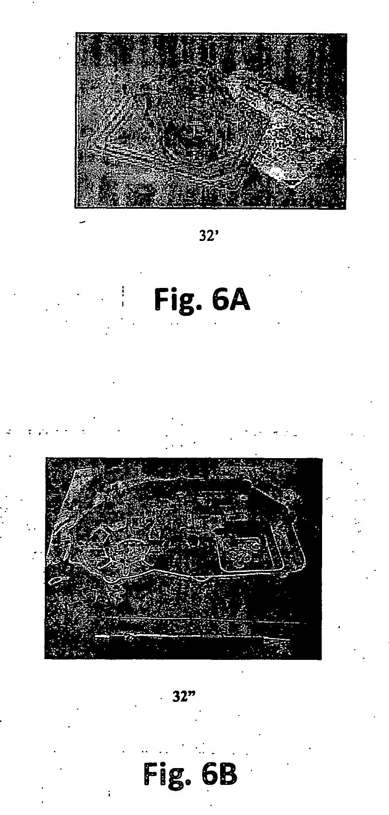

Referring now to **FIGs. 5A** to **5E****,** the method 60 of fabrication of the kit 30 includes several steps. Referring in particular to **FIG. 5B****,** in a first fabrication step 62, CT/MRI data 63 of a patient is created using a CT or MRI scanner. In a second fabrication step 64, the form of the patient-specific skull fragment is engineered from CT/MRI data, including inputs and modifications requested by the surgeon (e.g. consideration of muscle placement which may change the geometry of the fragment, etc). In a third fabrication step 66, the deep draw form 68 is designed using the 3D data 63 of the form of the patient-specific skull fragment 10. Optionally recesses 70 for accommodation of standard containers of bone cement 40a, 40b, a spatula or mixer 42, are included in the data so as to create a blister pack kit 30 including all operation-specific components and tools needed to complete the operation. Referring to **FIG. 5C****,** in a fourth fabrication step 72, the custom deep draw form 68 is fabricated by, for example, laser sintering, described in **FIG. 5D** using methods known in the art and/or practiced by Inspire AG, Switzerland. Optionally, this form 68 is made as an insert to a thermoforming mold which includes a form for creating the above-mentioned recesses 70. Referring now to **FIG. 5E****,** in a fifth fabrication step 74, the kneading form 32 is fabricated through, e.g., thermoforming a PP sheet, optionally, together with additional compartments for other components needed to complete the operation. See **FIGs. 6A** and **6B** for examples of thermoformed sheets 32' and 32". In a sixth fabrication step 76, the kneading form 32 is sterilized which comprises a sealing and sterilization of the PP kneading form. Examples of PP forms shaped by thermoforming are provided in **FIGs. 6A** and **6B****.**

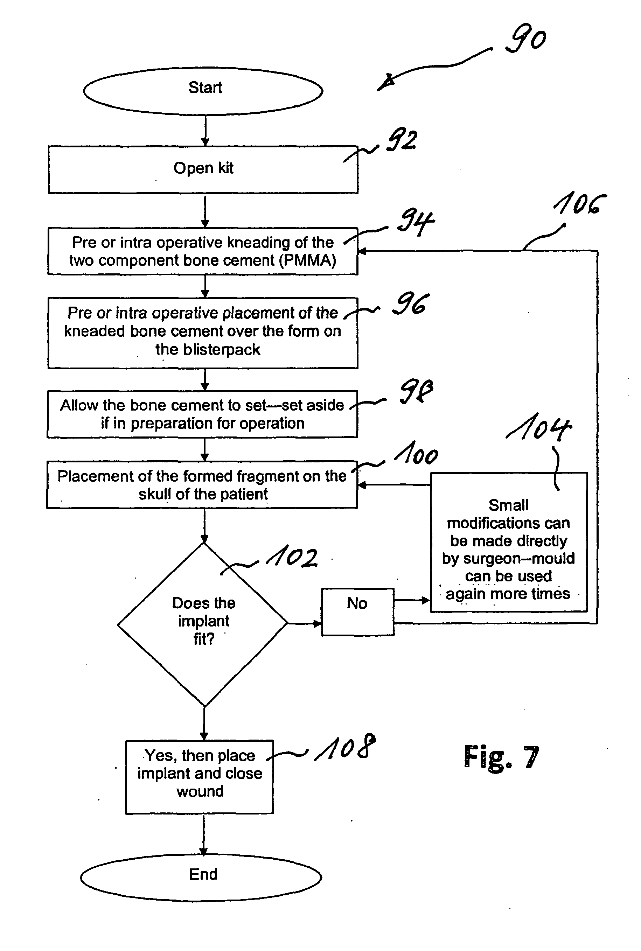

Referring now to **FIGs. 7** and **8A** and **8B,** the method of use 90 of the kit 30 includes several steps. In a first step 92, the kit is opened, exposing the patient-specific thermoformed form. In a second step 94, the two components 40a, 40b of the bone cement (PMMA) are mixed and kneaded to the desired consistency (see **FIGs. 2A-2B**). In a third step 96, the kneaded bone cement 98 is placed over the patient-specific thermoformed form 32 in order to shape and thus form the implant 16. In a fourth step 98, the kneaded bone cement 98 is allowed to set. In a fifth step 100, the formed implant 16 is placed on the skull 12 of the patient in the only position where it will be fixed (in the pre-surgical case this step can be proved with a laser sintered model of the skull). In a sixth step 102, the surgeon checks if the implant 16 properly fits in place. If not, in a seventh step 104, the surgeon evaluates whether to attempt to modify the replacement fragment. In an eighth step 106, if the surgeon cannot make satisfactory modifications, then the method returns to the first or second step 92, 94, and a new implant 16 is made. In a ninth step 108, the implant 16 is fixed in place, thus closing the wound.

Skilled artisans will appreciate that the configurations depicted in the figures are provided for representative and convenient illustration and that many other configurations may be alternatively, conjunctively and/or sequentially employed to produce substantially the same result. The present invention may be described herein in terms of functional block components and various processing steps. It should be appreciated that such functional blocks may be realized by any number of hardware and/or software components configured to perform the specified functions. For example, the present invention may employ various integrated circuit components, e.g., memory elements, processing elements, logic elements, data structures, and/or the like, which may carry out a variety of functions under the control of one or more microprocessors or other control devices. Similarly, the software elements of the present invention may be implemented with any programming or scripting language and/or any programming or scripting language now known or hereafter derived or otherwise described in the art, with the various algorithms being implemented with any combination of data structures, objects, processes, routines or other programming elements.

It should be appreciated that the particular implementations shown and described herein are representative of the invention and its best mode and are not intended to limit the scope of the present invention in any way. Indeed, for the sake of brevity, conventional data networking, application development and other functional aspects of the systems (and components of the individual operating components of the systems) may not be described in detail herein. Furthermore, the connecting lines shown in the various figures contained herein are intended to represent exemplary functional relationships and/or physical couplings between various elements. It should be noted that many alternative or additional functional relationships or physical connections may be present in a practical system.

As will be appreciated by those skilled in the art, the present invention may be embodied as a method, a system, a device, and/or a computer program product.

Moreover, the system contemplates the use, sale and/or distribution of any goods, services or information having similar functionality described herein.

The specification and figures are to be considered in an illustrative manner, rather than a restrictive one and all modifications described herein are intended to be included within the scope of the invention claimed, even if such is not specifically claimed at the filing of the application. Accordingly, the scope of the invention should be determined by the claims appended hereto or later amended or added, and their legal equivalents rather than by merely the examples described above. For instance, steps recited in any method or process claims may be executed in any order and are not limited to the specific order presented in any claim. Further, the elements and/or components recited in any apparatus claims may be assembled or otherwise operationally configured in a variety of permutations to produce substantially the same result as the present invention. Consequently, the invention is not limited to the specific configuration recited in the claims.

The direct benefits of the invention are reduced OR time, an optimal form of the implant and a correspondingly better fit, the use of tried and proven bone cement which allows for modification using common surgical tools, and a corresponding reduction in healthcare costs.

In an advantage of the invention, the time required to perform a surgical intervention is reduced, particularly the time required for the replacement of a three dimensional bone fragment 10 with a prostheses 16, 20.

In another advantage, the operation can be performed quickly, using bone cement, a material which has been tested in many applications and has been known and trusted for years. Using bone cement, if a correction to the form of the implant 16 is required, this can be done by the surgeon or an assistant using readily available surgical tools. See **FIG. 2C.**

In another advantage, the distraction of a surgeon's attention from the patient is reduced during the time that the surgeon would otherwise be intra-operatively forming a replacement cranial fragment 16.

In another advantage, the invention minimizes or eliminates the down-time associated with hardening or setting of the bone cement from which the replacement cranial fragment 16 is formed.

In another advantage, the invention minimizes the costs associated with a neurosurgical operation.

In another advantage, the delivery time from receipt of the CT/MRI data is reduced to about one to three weeks.

Benefits, other advantages and solutions mentioned herein are not to be construed as critical, required or essential features or components of any or all the claims.

As used herein, the terms "comprises", "comprising", or any variation thereof, are intended to refer to a non-exclusive listing of elements, such that any process, method, article, composition or apparatus of the invention that comprises a list of elements does not include only those elements recited, but may also include other elements described in this specification. The use of the term "consisting" or "consisting of" or "consisting essentially of" is not intended to limit the scope of the invention to the enumerated elements named thereafter, unless otherwise indicated. Other combinations and/or modifications of the above-described elements, materials or structures used in the practice of the present invention may be varied or otherwise adapted by the skilled artisan to other design without departing from the scope of the invention.

Other characteristics and modes of execution of the invention are described in the appended claims.

The copyrights in any appendix hereto are owned by the Applicant(s) or their assignee and, with respect to express Licensees of the rights defined in one or more claims herein, no implied license is granted herein to use the invention as defined in the remaining claims. Further, vis-à-vis third parties, including the public, no express or implied license is granted to reproduce, prepare derivative works, distribute copies, display, or otherwise use this patent specification, inclusive of the appendix hereto and any computer program comprised therein, except as an appendix to a patent issuing hereon.

Multiple variations and modifications are possible in the embodiments of the invention described here. Although certain illustrative embodiments of the invention have been shown and described here, a wide range of modifications, changes, and substitutions is contemplated in the foregoing disclosure. While the above description contains many specifics, these should not be construed as limitations on the scope of the invention, but rather as exemplifications of one or another preferred embodiment thereof. In some instances, some features of the present invention may be employed without a corresponding use of the other features. Accordingly, it is appropriated that the foregoing description be construed broadly and understood as being given by way of illustration and example only, the scope of the invention being limited only by the claims which ultimately issue in this application.

## Claims

1. A kit (30) comprising a base body (32) which has a sterilized mould recess (34) for receiving and shaping a not yet cross-linked and/or not yet hardened kneadable mass (40a, 40b); and a removable sealing element (46), **characterized in that** said mould recess (34) has a patient-specific three-dimensional negative surface contour of at least a part of a patient-specific body part structure (10) such as bone, cartilage or teeth; and said removable sealing element (46) hermetically seals said sterilized mould recess (34).

2. The kit according to claim 1, wherein said base body (32) has a sterilized kneading recess (44) for receiving said not yet hardened or cross-linked mass (40a, 40b) to be kneaded; and a removable sealing element (46) hermetically sealing said sterilized kneading recess (44).

3. The kit according to claim 1 or 2, wherein said base body (32) has a sterilized storage recess (70) in which a first sterilized kneadable mass component (40a) and a second sterilized kneadable mass component (40b) for preparing said kneadable mass are provided separately from each other; and a removable sealing element (44) hermetically sealing said sterilized storage recess (70) .

4. The kit according to any one of claims 1 to 3, wherein said base body (32) has a sterilized storage recess (70) in which a sterilized mixing and/or kneading tool (42) for mixing and/or kneading said not yet cross-linked or not yet hardened mass (40a, 40b) is provided; and a sealing element (46) hermetically sealing said sterilized storage recess (70).

5. The kit according to any one of claims 1 to 4, wherein said base body (32) is a thermoformed sheet.

6. The kit according to any one of claims 1 to 5, wherein a sealing element (46) is a foil removably attached to said base body (32) and hermetically sealing the recesses (34, 44, 70) of said base body.

7. The kit according to any one of claims 1 to 6, wherein a sealing element (46) is a hermetically sealed bag enclosing said base body (32).

8. The kit according to claims 1 to 7, wherein at least the sterilized surface inside said mould recess (34) and preferably inside said kneading recess (44) is made of an apolar polymer such as polypropylene (PP), polyethylene (PE) or polystyrene (PS) .

9. The kit according to any one of claims 5 to 8, wherein said thermoformed sheet (32) is made of an apolar polymer such as polypropylene (PP), polyethylene (PE) or polystyrene (PS).

10. A method of fabricating a kit (30) as defined in any one of claims 1 to 9, comprising the following steps:
a) three-dimensionally scanning a patient's body part structure (10) which is to be at least partially replaced and storing said tomographical data (63);
b) manufacturing a base (32) body having at least one recess (34) with a patient-specific three-dimensional negative surface contour based on said tomographical data (63);
c) sterilizing the at least one recess (34) of said base body (32) and hermetically sealing it with a removable sealing element (46) .

11. The method according to claim 10, wherein step b) consists of manufacturing said base body (32) using an additive (layer-by-layer) process such as laser sintering and providing said base body (32) as the base body of the kit.

12. The method according to claim 10, wherein step b) comprises a step b1) for manufacturing an intermediate body (68) using an additive (layer-by-layer) process such as laser sintering; and a step b2) for using said intermediate body (68) as a mould in a thermoforming process such as deep-drawing for thermoforming a sheet material into a thermoformed sheet (32, 32', 32'') and providing said thermoformed sheet as the base body of the kit.

13. A method of using a kit (30) according to any one of claims 1 to 9, comprising the following steps:
- removing the sealing element (46) from the base body (32) in a sterilized environment, thus exposing the sterilized mould recess (34) ;
- preparing a kneadable and cross-linkable and/or hardenable (settable) mass (40a, 40b);
- placing the prepared mass (40a, 40b) into the sterilized mould recess (34), thus shaping at least a part of the surface of said mass (40a, 40b) in accordance with a patient-specific body part structure (10);
- allowing the shaped mass to at least partially cross-link and/or harden; and
- removing the at least partially cross-linked and/or hardened shaped mass (40a, 40b) from said recess (34).

## Patentansprüche

1. Kit (30), umfassend einen Grundkörper (32), der eine sterilisierte Formvertiefung (34) zum Aufnehmen und Formen einer noch nicht vernetzten und/oder noch nicht ausgehärteten knetbaren Masse (40a, 40b) sowie ein entfernbares Abdichtelement (46) aufweist, **dadurch gekennzeichnet, dass** die Formvertiefung (34) einen patientenspezifischen dreidimensionalen negativen Flächenumriss von mindestens einem Teil einer patientenspezifischen Körperteilstruktur (10) wie Knochen, Knorpel oder Zähne aufweist und das entfernbare Abdichtelement (46) die sterilisierte Formvertiefung (34) hermetisch abdichtet.

2. Kit nach Anspruch 1, wobei der Grundkörper (32) eine sterilisierte Knetvertiefung (44) zum Aufnehmen der noch nicht ausgehärteten oder vernetzten zu knetenden Masse (40a, 40b) aufweist; sowie ein entfernbares Abdichtelement (46), das die sterilisierte Knetvertiefung (44) hermetisch abdichtet.

3. Kit nach Anspruch 1 oder 2, wobei der Grundkörper (32) eine sterilisierte Aufbewahrungsvertiefung (70) aufweist, in der ein erster sterilisierter knetbarer Massebestandteil (40a) und ein zweiter sterilisierter knetbarer Massebestandteil (40b) zum Herstellen der knetbaren Masse getrennt voneinander vorgesehen sind; sowie ein entfernbares Abdichtelement (44), das die sterilisierte Aufbewahrungsvertiefung (70) hermetisch abdichtet.

4. Kit nach einem der Ansprüchen 1 bis 3, wobei der Grundkörper (32) eine sterilisierte Aufbewahrungsvertiefung (70) aufweist, in der ein sterilisiertes Misch- und/oder Knetwerkzeug (42) zum Mischen und/oder Kneten der noch nicht vernetzten oder noch nicht ausgehärteten Masse (40a, 40b) vorgesehen ist; sowie ein entfernbares Abdichtelement (46), das die sterilisierte Aufbewahrungsvertiefung (70) hermetisch abdichtet.

5. Kit nach einem der Ansprüche 1 bis 4, wobei es sich bei dem Grundkörper (32) um eine thermogeformte Folie handelt.

6. Kit nach einem der Ansprüche 1 bis 5, wobei es sich bei einem Abdichtelement (46) um eine Folie handelt, die entfernbar an dem Grundkörper (32) befestigt ist und die Vertiefungen (34, 44, 70) des Grundkörpers hermetisch abdichtet.

7. Kit nach einem der Ansprüche 1 bis 6, wobei es sich bei einem Abdichtelement (46) um einen hermetisch verschlossenen Beutel handelt, der den Grundkörper (32) umschließt.

8. Kit nach einem der Ansprüche 1 bis 7, wobei zumindest die sterilisierte Fläche in der Formvertiefung (34) und vorzugsweise in der Knetvertiefung (44) aus einem unpolaren Polymer wie Polypropylen (PP), Polyethylen (PE) oder Polystyrol (PS) gefertigt ist.

9. Kit nach einem der Ansprüche 5 bis 8, wobei die thermogeformte Folie (32) aus einem unpolaren Polymer wie Polypropylen (PP), Polyethylen (PE) oder Polystyrol (PS) gefertigt ist.

10. Verfahren zum Herstellen eines Kits (30) nach einem der Ansprüche 1 bis 9, das die folgenden Schritte umfasst:
a) dreidimensionales Scannen einer Körperteilstruktur (10) eines Patienten, die zumindest teilweise ersetzt werden soll, und Speichern der tomographischen Daten (63);
b) Herstellen eines Grundkörpers (32) mit mindestens einer Vertiefung (34) mit einem patientenspezifischen dreidimensionalen negativen Flächenumriss auf der Grundlage der tomographischen Daten (63);
c) Sterilisieren der mindestens einen Vertiefung (34) des Grundkörpers (32) und hermetisches Abdichten derselben mit einem entfernbaren Abdichtelement (46).

11. Verfahren nach Anspruch 10, wobei Schritt b) aus dem Fertigen des Grundkörpers (32) unter Verwendung eines additiven (schichtweise erfolgenden) Prozesses wie Lasersintern und dem Bereitstellen des Grundkörpers (32) als Grundkörper des Kits besteht.

12. Verfahren nach Anspruch 10, wobei Schritt b) einen Schritt b1) zum Fertigen eines Zwischenkörpers (68) unter Verwendung eines additiven (schichtweise erfolgenden) Prozesses wie Lasersintern; sowie einen Schritt b2) zum Verwenden des Zwischenkörpers (68) als eine Form in einem Thermoformprozess wie Tiefziehen zum Thermoformen eines Folienmaterials zu einer thermogeformten Folie (32, 32', 32'') und Bereitstellen der thermogeformten Folie als Grundkörper des Kits umfasst.

13. Verfahren zum Verwenden eines Kits (30) nach einem der Ansprüche 1 bis 9, das die folgenden Schritte umfasst:
- Entfernen des Abdichtelements (46) von dem Grundkörper (32) in einer sterilen Umgebung, wodurch die sterilisierte Formvertiefung (34) freigelegt wird;
- Herstellen einer knetbaren und vernetzbaren und/oder aushärtbaren (abbindbaren) Masse (40a, 40b);
- Platzieren der hergestellten Masse (40a, 40b) in der sterilisierten Formvertiefung (34), wodurch zumindest ein Teil der Fläche der Masse (40a, 40b) entsprechend einer patientenspezifischen Körperteilstruktur (10) geformt wird;
- Zulassen, dass die geformte Masse zumindest teilweise vernetzt und/oder aushärtet; und
- Herausnehmen der zumindest teilweise vernetzten und/oder ausgehärteten geformten Masse (40a, 40b) aus der Vertiefung (34).

## Revendications

1. Coffret (30) comprenant un corps de base (32) qui a une cavité de moulage stérilisée (34) pour recevoir et former une masse malaxable non encore réticulée et/ou non encore durcie (40a, 40b) ; et un élément de scellement détachable (46), **caractérisé par le fait que** ladite cavité de moulage (34) a un contour de surface négatif tridimensionnel, spécifique au patient, d'au moins une partie d'une structure de partie de corps spécifique au patient (10) telle qu'un os, un cartilage ou des dents ; et ledit élément de scellement détachable (46) scelle de manière hermétique ladite cavité de moulage stérilisée (34) .

2. Coffret selon la revendication 1, dans lequel ledit corps de base (32) comprend une cavité de malaxage stérilisée (44) pour recevoir ladite masse non encore durcie ou réticulée (40a, 40b) devant être malaxée ; et un élément de scellement détachable (46) scellant de manière hermétique ladite cavité de malaxage stérilisée (44).

3. Coffret selon la revendication 1 ou 2, dans lequel ledit corps de base (32) comprend une cavité de stockage stérilisée (70) dans laquelle un premier composant de masse malaxable stérilisé (40a) et un second composant de masse malaxable stérilisé (40b) pour préparer ladite masse malaxable sont disposés séparément l'un de l'autre ; et un élément de scellement détachable (44) scellant de manière hermétique ladite cavité de stockage stérilisée (70) .

4. Coffret selon l'une quelconque des revendications 1 à 3, dans lequel ledit corps de base (32) comprend une cavité de stockage stérilisée (70) dans laquelle est disposé un outil de mélange et/ou de malaxage stérilisé (42) pour mélanger et/ou malaxer ladite masse non encore réticulée ou non encore durcie (40a, 40b) ; et un élément de scellement (46) scellant de manière hermétique ladite cavité de stockage stérilisée (70).

5. Coffret selon l'une quelconque des revendications 1 à 4, dans lequel ledit corps de base (32) est une feuille thermoformée.

6. Coffret selon l'une quelconque des revendications 1 à 5, dans lequel un élément de scellement (46) est une feuille fixée de façon détachable audit corps de base (32) et scellant de manière hermétique les cavités (34, 44, 70) dudit corps de base.

7. Coffret selon l'une quelconque des revendications 1 à 6, dans lequel un élément de scellement (46) est un sac scellé de manière hermétique renfermant ledit corps de base (32).

8. Coffret selon les revendications 1 à 7, dans lequel au moins la surface stérilisée à l'intérieur de ladite cavité de moulage (34) et, de préférence, à l'intérieur de ladite cavité de malaxage (44) est faite d'un polymère apolaire tel que le polypropylène (PP), le polyéthylène (PE) ou le polystyrène (PS).

9. Coffret selon l'une quelconque des revendications 5 à 8, dans lequel ladite feuille thermoformée (32) est faite d'un polymère apolaire tel que le polypropylène (PP), le polyéthylène (PE) ou le polystyrène (PS).

10. Procédé de fabrication d'un coffret (30) tel que défini à l'une quelconque des revendications 1 à 9, comprenant les étapes suivantes :
a) balayer en trois dimensions une structure de partie de corps (10) d'un patient, laquelle doit être au moins partiellement remplacée, et stocker lesdites données tomographiques (63) ;
b) fabriquer un corps de base (32) ayant au moins une cavité (34) avec un contour de surface négatif tridimensionnel spécifique au patient, sur la base desdites données tomographiques (63) ;
c) stériliser l'au moins une cavité (34) dudit corps de base (32) et sceller de manière hermétique celle-ci avec un élément de scellement détachable (46).

11. Procédé selon la revendication 10, dans lequel l'étape b) consiste en la fabrication dudit corps de base (32) à l'aide d'une technique des procédés additifs (couche par couche), tel qu'un frittage laser, et la fourniture dudit corps de base (32) en tant que corps de base du coffret.

12. Procédé selon la revendication 10, dans lequel l'étape b) comprend une étape b1) pour fabriquer un corps intermédiaire (68) à l'aide d'une technique des procédés additifs (couche par couche), tel qu'un frittage laser ; et une étape b2) pour utiliser ledit corps intermédiaire (68) comme moule dans un procédé de thermoformage, tel qu'un formage profond, pour thermoformer une matière de feuille en une feuille thermoformée (32, 32', 32" ) et fournir ladite feuille thermoformée en tant que corps de base du coffret.

13. Procédé d'utilisation d'un coffret (30) selon l'une quelconque des revendications 1 à 9, comprenant les étapes suivantes :
- retirer l'élément de scellement (46) du corps de base (32) dans un environnement stérilisé, exposant ainsi la cavité de moulage stérilisée (34) ;
- préparer une masse malaxable et réticulable et/ou durcissable (apte à subir une prise) (40a, 40b) ;
- placer la masse préparée (40a, 40b) dans la cavité de moulage stérilisée (34), mettant ainsi en forme au moins une partie de la surface de ladite masse (40a, 40b) conformément à une structure de partie de corps spécifique au patient (10) ;
- laisser la masse mise en forme se réticuler et/ou durcir au moins partiellement ; et
- retirer la masse mise en forme, au moins partiellement réticulée et/ou durcie (40a, 40b), de ladite cavité (34) .
